# EUROPÄISCHE PATENTANMELDUNG

(11) **EP 1 435 250 A1**
(43) Veröffentlichungstag der Anmeldung: **07.07.2004**
(21) Anmeldenummer: 03024923.9
(22) Anmeldetag: 29.10.2003
(51) Int. Cl.: A61M 1/00, A61M 3/02

(54) **Medizinisches Spül- und Sauggerät**

(30) Priorität: 06.11.2002 DE 10251598
(71) Anmelder: A.M.I. (Agency for Medical Innovations GmbH), 6840 Götzis (AT)
(72) Erfinder: Steffen, Arnold, Dr., 6700 Bludenz (AT)
(74) Vertreter: Riebling, Peter, Dr.-Ing.

(57) **Zusammenfassung**

Es wird ein medizinisches Spül- und Sauggerät zum Spülen und Absaugen von Körperflächen in einem Operationsgebiet beschrieben, das aus einer Spül- und Absauglanze und einer damit verbundenen Ventilanordnung mit umschaltbaren Ventilen besteht, die je nach Ventilstellung eine Spülflüssigkeit der Spül- und Absauglanze zuführen oder diese in Absaugfunktion schalten. Um das Gerät besser handhaben zu können sieht die Erfindung vor, dass alle Funktionsteile in einem handhabbaren Gerät vereinigt sind.

## Beschreibung

Die Erfindung betrifft ein medizinisches Spül- und Sauggerät nach dem Oberbegriff des Patentanspruchs 1.

Medizinische Spül- und Sauggeräte werden zur Spülung von Wundflächen verwendet, wobei diese Wundflächen im äußeren Bereich, aber auch im Innenraum des Körpers liegen können. Geht es um die Spülung und Absaugung von Wundflächen mit Operationsgeräten im Innenraum des Körpers, dann wird an das medizinische Spül- und Sauggerät ein endoskopisches Spül-Saugrohr angeschlossen.

Es ist ein eingangs genanntes Spül- und Sauggerät bekannt geworden, welches für den Einmalgebrauch geeignet ist.

An einer Lanze, die sowohl als Spül- als auch als Absaugkanal verwendet wird, ist eine Ventilanordnung angeschlossen, an die zwei unterschiedliche Schläuche ansetzen. Wird das eine Ventil betätigt, dann kann mit einem entsprechenden Spülschlauch Spülflüssigkeit über die Lanze in die Körperöffnung eingeführt werden. Nach Schließen des einen Ventils kann das andere Ventil geöffnet werden und die in die Körperöffnung eingespülte Flüssigkeit wird wieder abgesaugt.

Nachteil dieser Anordnung ist jedoch, dass die Lanze sowohl zur Spülung als auch zur Absaugung verwendet wird, was mit einer Verunreinigung des Operationsgebietes verbunden ist. Wird nämlich zunächst abgesaugt und danach folgend wieder gespült, so werden mit dem ersten Spülvorgang die abgesaugten Partikel zunächst wieder in den Wundbereich hineinbefördert, um dann wieder abgesaugt zu werden.

Weiterer Nachteil dieser Anordnung ist, dass die Ventilanordnung nicht betriebssicher zu bedienen ist. Bei einer Fehlbedienung ist es nicht ausgeschlossen, dass sowohl das Spülventil als auch das Absaugventil gleichzeitig betätigt werden, wodurch der Spülstrom sofort im Ventilkörper wieder abgesaugt wird. Eine Spülung der Körperfläche findet nicht statt.

Weiterer Nachteil des bekannten medizinischen Spül- und Sauggerätes ist, dass ein relativ großer Apparateaufwand betrieben werden muss. In einem separaten Standgehäuse ist eine doppelwirkende Pumpe vorhanden, mit der sowohl der Spülvorgang als auch der Absaugvorgang mit großem Strömungsvolumen durchgeführt werden. Daher bedarf es einer eigenen Maschine, die im Operationssaal separat aufgestellt wird. Ein solches medizinisches Spül- und Sauggerät hat sich für das Spülen und Absaugen großer Flüssigkeitsvolumina bewährt. Kommt es hingegen darauf an, nur kleine Wundflächen oder nur kleine Flüssigkeitsvolumen zu spülen, dann ist der Aufwand, der mit der bekannten Anordnung betrieben wird, zu groß. Es handelt sich ja um eine Anordnung für den Einmalgebrauch, nach deren Verwendung die gesamte Ventileinheit zusammen mit der Absauglanze und allen Zufuhrschläuchen ausgewechselt werden muss.

Der Erfindung liegt deshalb die Aufgabe zugrunde, ein medizinisches Spül- und Absauggerät der eingangs genannten Art so weiter zu bilden, dass mit wesentlich geringerem Maschinenaufwand und bei kostengünstigerer Herstellung ein verbesserter Effekt erzielt wird.

Zur Lösung der gestellten Aufgabe ist die Erfindung dadurch gekennzeichnet, dass an einer handbetätigten Saug- und Spülpumpe jeweils mindestens ein Spülbehälter und getrennt davon ein Auffangbehälter angeordnet sind.

Mit der gegebenen technischen Lehre ergibt sich der wesentliche Vorteil, dass nun so zu sagen alle Bestandteile in einem handhaltbaren Gerät enthalten sind. Die erfindungsgemäße Saug- und Spülpumpe enthält die zur Erzeugung eines Spülflüssigkeitsstromes erforderliche Spülpumpe und gleichzeitig auch getrennt davon eine für das Absaugen aus dem Wundgebiet notwendige Absaugpumpe.

Jeder Pumpe ist ein eigener Behälter zugeordnet. Damit ergibt sich der Vorteil, dass die Spülflüssigkeit aus dem Spülbehälter von der Spülpumpe angesaugt und über das Spül-Saugrohr in das Wundgebiet gebracht wird und dass nach dem Umstellen der Handbetätigung an der Saug-Druckpumpe mit einer weiteren handbetätigten Saugpumpe die in das Wundgebiet eingespülte Flüssigkeit wieder abgesaugt wird und in den an der Saug-Druckpumpe angeordneten Auffangbehälter eingeleitet wird.

In einer bevorzugten Ausgestaltung der Erfindung ist es vorgesehen, dass mindestens der Auffangbehälter abnehmbar und damit auswechselbar ausgebildet ist.

In einer weiteren Ausgestaltung der Erfindung kann es auch vorgesehen sein, dass auch der Spülbehälter abnehmbar und auswechselbar an der Saug- und Druckpumpe angeordnet ist. Hierauf ist die Erfindung jedoch nicht beschränkt. Es kann auch vorgesehen sein, dass der Spülbehälter unmittelbar in der Sau-Druckpumpe integriert ist, um mit dieser eine Einheit zu bilden.

Erfindungsgemäß wird nämlich die Saug- und Druckpumpe ebenfalls für den Einmalgebrauch ausgerichtet, d. h. nach einmaligem Gebrauch wird sie entsorgt.

Die Abnehmbarkeit des Auffangbehälters hat den Vorteil, dass die aus dem Wundgebiet abgesaugten Gewebeteilchen und mit Keimen belastete Flüssigkeiten und dergleichen leicht untersucht werden können, wenn der Auffangbehälter nach dem Abschrauben von der Saug-Druckpumpe verschlossen und der Inhalt in einem entsprechenden Labor untersucht wird.

Die Erfindung sieht in einer ersten bevorzugten Ausführungsform deshalb vor, dass sowohl für das Absaugen eine eigene Absaugpumpe und für das Spülen eine weitere Spülpumpe vorhanden sind. Diese beiden Pumpen werden wechselseitig in Betrieb genommen und zwar durch einen Schieber, der an dem handbetätigten Hebel der Saug-Druckpumpe angeordnet ist.

Je nach der Schieberstellung an diesem Hebel wird deshalb nur entweder die Spülpumpe oder die Saugpumpe betätigt.

Die Erfindung ist jedoch nicht auf die Anordnung von zwei voneinander getrennten Pumpen beschränkt. In einer anderen Ausgestaltung der Erfindung ist es vorgesehen, dass die Spülpumpe entfällt und dass der Spülbehälter unter Überdruck steht und die Flüssigkeit deshalb nur unter der Einwirkung eines handbetätigten Ventils in den Operationsbereich gebracht wird.

In einer weiteren bevorzugten Ausgestaltung der Erfindung ist es vorgesehen, dass das Spül-Saugrohr zwei getrennt voneinander angeordnete Kanäle aufweist. Der eine, größer kalibrierte Kanal ist zum Absaugen auch größerer Wund- und Gewebeteilchen geeignet, während der andere, kleiner kalibrierte Kanal für die Zuführung der Spülflüssigkeit in das Operationsgebiet vorgesehen ist. Damit besteht der Vorteil, dass die belasteten Keime nicht mehr in den Spülkreislauf kommen können, so wie dies bei dem Stand der Technik der Fall war.

Der Erfindungsgegenstand der vorliegenden Erfindung ergibt sich nicht nur aus dem Gegenstand der einzelnen Patentansprüche, sondern auch aus der Kombination der einzelnen Patentansprüche untereinander.

Alle in den Unterlagen, einschließlich der Zusammenfassung offenbarten Angaben und Merkmale, insbesondere die in den Zeichnungen dargestellte räumliche Ausbildung, werden als erfindungswesentlich beansprucht, soweit sie einzeln oder in Kombination gegenüber dem Stand der Technik neu sind.

Im Folgenden wird die Erfindung anhand von lediglich einen Ausführungsweg darstellenden Zeichnungen näher erläutert. Hierbei gehen aus den Zeichnungen und ihrer Beschreibung weitere erfindungswesentliche Merkmale und Vorteile der Erfindung hervor.

Es zeigen:
- Figur 1:: Schnitt durch eine Saug-Druckpumpe nach der Erfindung;
- Figur 2:: eine teilweise zerlegte Saug-Druckpumpe nach Figur 1;
- Figur 3:: Schnitt durch die Saugpumpe;
- Figur 4:: Stirnansicht der Saugpumpe nach Figur 3;
- Figur 5:: Schnitt durch die Spülpumpe;
- Figur 6:: Stirnansicht der Spülpumpe nach Figur 5;
- Figur 7:: Schnitt durch den Hebel;
- Figur 8:: Draufsicht auf den Hebel nach Figur 7;
- Figur 9:: Seitenansicht des Spül-Saugrohres;
- Figur 10:: Stirnansicht des Spül-Saugrohres nach Figur 9.

### Die Saug- und Druckpumpe 1

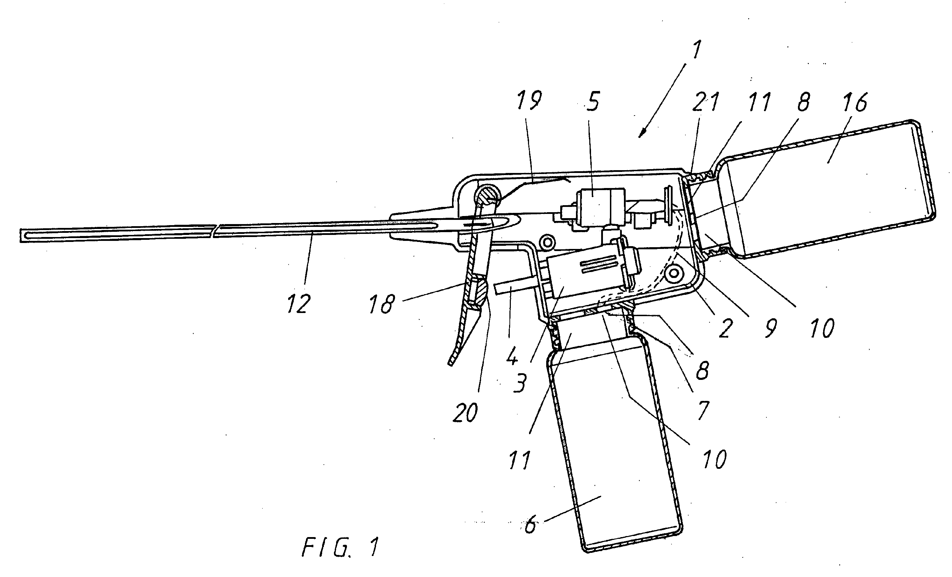

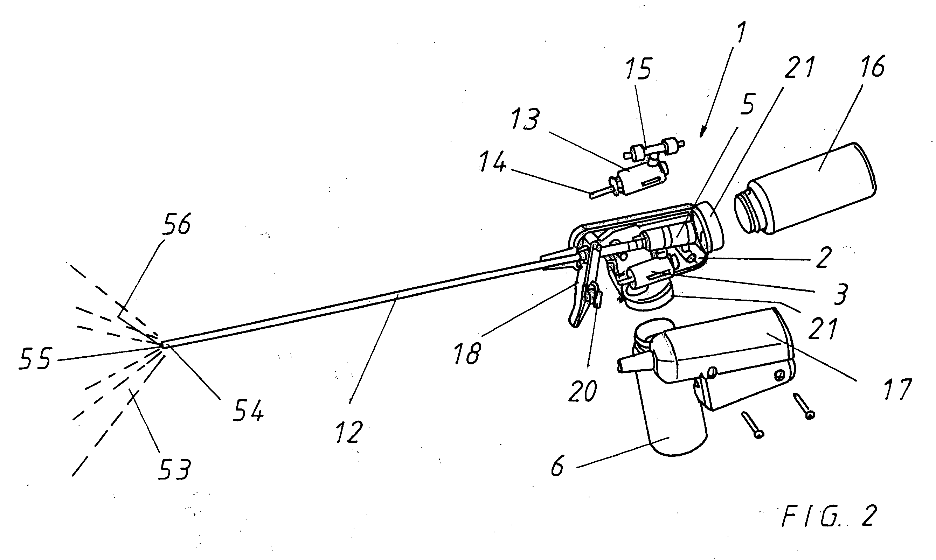

In den Figuren 1 und 2 besteht die erfindungsgemäße Saug-Druckpumpe 1 im Wesentlichen aus einen Gehäuse 2, welches aus zwei miteinander verschraubbaren Gehäuseschalen besteht, wobei lediglich die vordere Gehäuseschale 17 in Figur 2 dargestellt ist.

Im Innenraum des Gehäuses sind zwei unabhängig voneinander betätigbare Pumpen 3, 13 angeordnet, wobei die eine Pumpe als Saugpumpe 3 und die andere als Spülpumpe 13 ausgebildet ist.

An der Unterseite des Gehäuses 2 ist hierbei ein Ansatz 21 angeordnet, der ein Gewinde 7 trägt. Auf dieses Gewinde wird ein Auffangbehälter 6 aufgeschraubt, der eine obere Dichtung 8 trägt und der eine Öffnung 10 für die Einführung des nicht näher dargestellten Schlauches 9 trägt. Ferner ist ein Atmungsventil 11 angeordnet, so dass sichergestellt ist, dass wenn die Saug-Druckpumpe zur Seite gelegt wird, die in dem Auffangbehälter 6 lagernde Flüssigkeit nicht unbeabsichtigt austritt.

### Die Saugpumpe 3

Der Saugpumpe 3 ist der Ventilblock 5 zugeordnet; sie wird über einen Stößel 4 betätigt, der von einem mit der Feder 19 schwenkbar gelagerten Hebel 18 beaufschlagt ist. Die Saugpumpe 3 besteht im Wesentlichen aus einem Zylinder 28, indem federbelastet über die Feder 30 ein Kolben 29 verschiebbar angeordnet ist. Der Kolben 29 ist mit einem Stößel 4 verbunden, der von dem vorher erwähnten Hebel 18 betätigt wird.

Es handelt sich um eine einfach wirkende Pumpe, weil die Zylinderkammer über dem Anschluss 26 mit dem Ventilblock 5 verbunden ist. Der Ventilblock 5 besteht aus zwei getrennt voneinander arbeitenden Ventilkammern 22, 23.

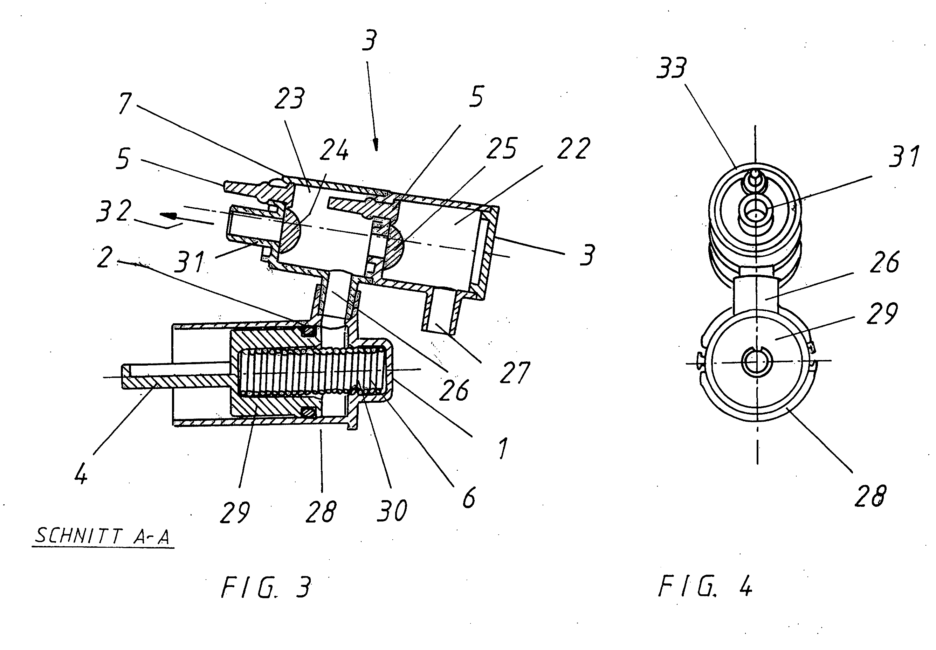

Wird der Stößel 4 in der in Figur 3 dargestellten Lage entlastet, dann bewegt sich der Kolben 29 nach links und saugt über den Anschluss 26 Flüssigkeit in die Zylinderkammer 1. Dadurch öffnet sich das in der Ventilkammer 23 angeordnete Ventil 24 und saugt in Gegenrichtung zur eingezeichneten Pfeilrichtung 32 Flüssigkeit in die Ventilkammer 23 ein. Gleichzeitig ist das Ventil 25 geschlossen. Nachdem durch den Saughub die Ventilkammer 23 gefüllt wurde, wird bei der nächsten Betätigung des Stößels 4 in Gegenrichtung das Ventil 25 geöffnet und die Ventilkammer 22 gefüllt. Das abgesaugte Material gelangt nun aus der Ventilkammer 22 über den Anschluss 27 und den Schlauch 9 in den Auffangbehälter 6.

Die dargestellten Ventile 24, 25 sind aus Silikon gefertigte Ventilklappen, die einen großen Querschnitt haben.

### Die Spülpumpe 13

Die Spülpumpe 13 ist im hinteren Bereich des Gehäuses angeordnet und der besseren Übersichtlichkeit halber in Figur 2 getrennt herausgezeichnet. Sie weist ebenfalls einen Stößel 14 auf, und an dem Zylinder sitzt oben ein Ventilblock 15 an, dessen Ausgang über einen nicht näher dargestellten Schlauch in die Öffnung 10 des Spülbehälters 16 eingeleitet ist.

Auch dieser Spülbehälter weist in der vorher erwähnten Weise eine Dichtung 8 mit einem Atmungsventil 11 auf und ist mit einem Gewinde auf dem Ansatz am Gehäuse der Saug-Druckpumpe 1 aufgeschraubt.

Mit der getrennten Anordnung einer Saugpumpe 3 zusammen mit einer Spülpumpe 13 besteht im Übrigen der Vorteil, dass der Saugpumpe 3 große Absaugquerschnitte zugeordnet werden können, so dass mit hohem Volumen abgesaugt werden kann und auch grobe Partikel mitgeführt werden können, wohingegen die Spülpumpe 13 nur einen relativ geringen Spülquerschnitt benötigt und entsprechend weniger Spülflüssigkeit in das Wundgebiet transportiert. Es muss deshalb nur die eine Pumpe auf große Querschnitte dimensioniert werden.

Aus diesem Grunde sind die Ventile im Ventilblock 15 der Spülpumpe 13 auch kleiner dimensioniert.

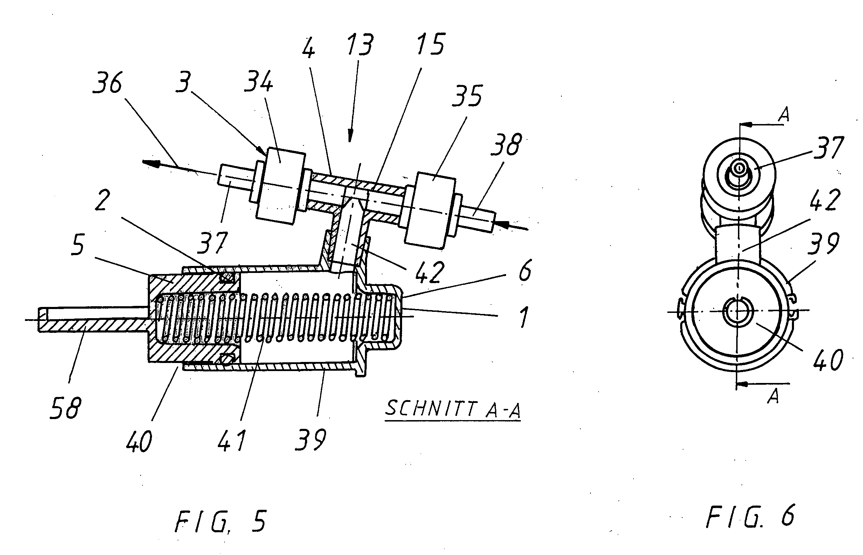

Die in Figur 5 und 6 dargestellt Spülpumpe 13 besteht demgemäss wiederum aus einem Zylinder 39, in dem über die Feder 41 belastet der Kolben 40 hin- und herbewegt wird. Der Kolben weist an seiner Vorderseite wiederum einen Stößel 58 auf.

Die Zylinderkammer ist über den Anschluss 42 mit dem Ventilblock 15 verbunden, der wiederum aus zwei Ventilkammern 34, 35 besteht. Es ist genau die gleiche Funktion gegeben, wie sie anhand der Saugpumpe 3 und den Figuren 3 und 4 erläutert wurde.

Bei einem entsprechenden Druckhub auf den Stößel 58 entgegen der Kraft der Feder 41 wird das Volumen der Zylinderkammer vermindert und das in der Zylinderkammer befindliche Spülvolumen wird in Pfeilrichtung 36 aus dem Anschluss 37 herausgespritzt, wobei das Ventil in der Ventilkammer 35 geschlossen und das Ventil in der Ventilkammer 34 geöffnet ist.

Bei dem nächsten Saughub wird hingegen das Ventil in der Ventilkammer 34 geschlossen und das Ventil in der Ventilkammer 35 geöffnet, so dass Flüssigkeit in der eingezeichneten Pfeilrichtung über den Anschluss 38 aus dem Spülbehälter 16 nachgesaugt wird.

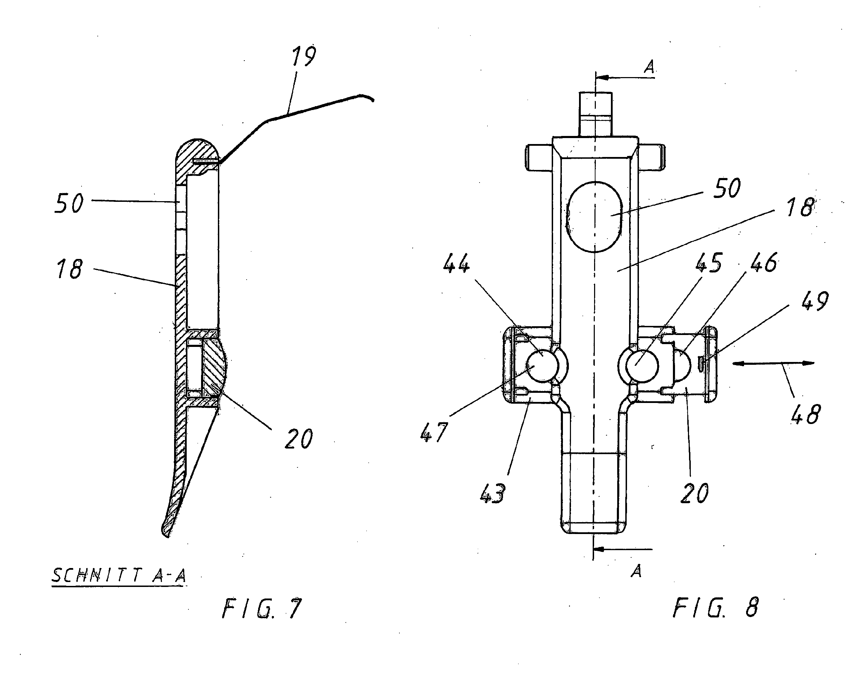

Die Figuren 7 und 8 zeigen den federbelasteten Hebel 18, der dafür sorgt, dass lediglich nur jeweils eine Pumpe, nämlich entweder die Saugpumpe 3 oder die Spülpumpe 13 betätigbar ist.
Dies wird dadurch erreicht, dass fest am Hebel 18 ein Querteil 53 angeordnet ist, in dem zwei voneinander beabstandete Bohrungen 44, 45 angeordnet sind.

In diesem Querteil 43 ist ein Schieber 20 verschiebbar angeordnet, der ebenfalls zwei zu den Bohrungen 44, 45 korrespondierende Bohrungen 46, 47 aufweist.

In der in Figur 8 dargestellten Verschiebungslage fluchtet daher die Bohrung 47 im Schieber mit der Bohrung 44 im Querteil des Hebels 18, so dass diese Bohrung frei durchgängig ist. In diese Bohrung greift nun beispielsweise der Stößel 4 der Saugpumpe 3 ein, so dass die Saupumpe bei einer Handbetätigung des Hebels 18 nicht betätigt werden kann.

In analoger Weise ist durch den Versatz der beiden Bohrung 45, 46 gerade die Bohrung 45 im Querteil durch den Schieber 20 verschlossen und der Stößel 58 der Spülpumpe 13 setzt auf den Verschluss der Bohrung 45 auf und wird demzufolge betätigt.

Wird hingegen der Schieber 20 in Pfeilrichtung 48 nach links verschoben, dann wird die Bohrung 44 verschlossen und die Bohrung 45 geöffnet. In dieser Verschiebungslage wird dann dementsprechend die Saugpumpe 3 in Betrieb gesetzt.

Durch die Anor4dnung des Schiebers 20 ist es daher ausgeschlossen, dass beiden Pumpen gleichzeitig betätigt werden, wie dies beim Stand der Technik der Fall war.

Im Schieber 20 ist im Übrigen noch eine Rastnase 49 angeordnet, um die beiden Verschiebungslagen zu arretieren.

Der Hebel 18 weist Im Übrigen eine größere Ausnehmung 50 auf, durch welche das Spül- und Saugrohr 12 hindurchgreift, welches im Folgenden näher beschrieben wird.

### Das Spül- und Saugrohr 12

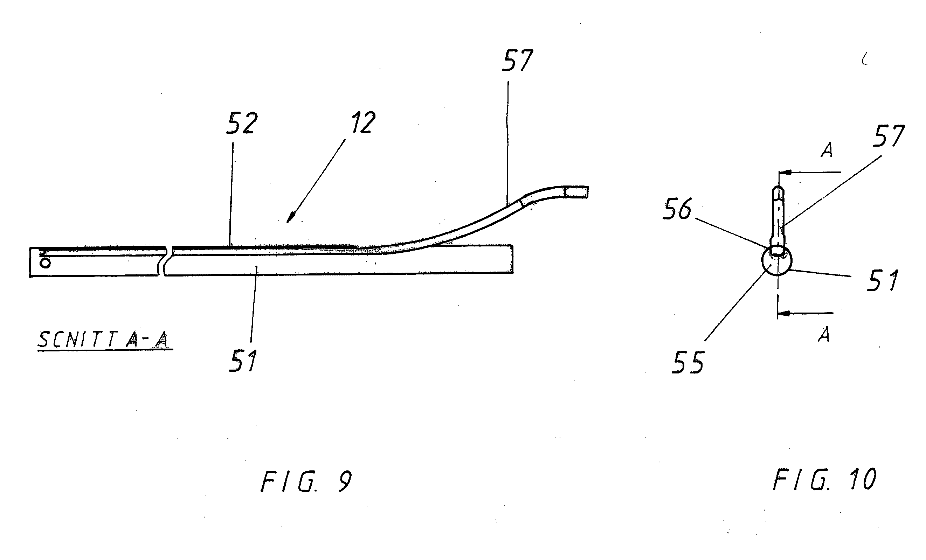

In einer bevorzugten Ausgestaltung nach den Figuren 9 und 10 ist das Spül-Saugrohr 12 zweiteilig ausgebildet und besteht aus einem größer kalibrierten Saugrohr 51, zu dem parallel und in Verbindung mit dem Saugrohr 51 ein Spülrohr 52 geführt ist. Die beiden Teile 51, 52 sind miteinander verbunden, wobei es verschiedene Möglichkeiten gibt:

In einer ersten Ausgestaltung kann es vorgesehen sein, dass das Spülrohr 52 im Innenraum des Saugrohres 51 geführt wird, so dass - gemäß Figur 10 - die Spüldüse 56 des Spülrohres 52 in der Ansaugbohrung 55 des Saugrohres 12 liegt.

In einer anderen Ausgestaltung der Erfindung kann es jedoch auch vorgesehen sein, dass das Spülrohr 52 außerhalb des Saugrohres 51 geführt ist, so dass dann auch die Spüldüse 56 außerhalb der Ansaugbohrung 55 im Spül-Saugrohr 12 liegt.

In Figur 2 ist die überlegene Spülwirkung der Spülpumpe 13 dargestellt. Es wird über die Spüldüse 56 ein Spülfächer 53 erzeugt, der großflächig über das Operationsgebiet verteilt werden kann. Über die vordere Ansaugbohrung 55, die groß kalibriert ist, werden auch große Gewebeteilchen und andere Teile abgesaugt. Sollte es hingegen zu einem Verschluss der Ansaugbohrung 55 kommen, so sind seitliche Entlastungsbohrung 54 an der Vorderseite des Spül-Saugrohres 12 angeordnet, um dennoch einen Absaugvorgang zu ermöglichen.

Wenn man die Spüldüse 56 im vorderen Bereich der Ansaugbohrung 55 führt und diese auch noch anschrägt, dann ergibt sich ein großflächiger Spülfächer 53, mit dem optimal das zu reinigende Wundgebiet gespült werden kann.

Vorteil der erfindungsgemäßen Saug-Druckpumpe ist, dass es sich um relativ kostengünstige Teile handelt, die durchaus für eine Einmalgebrauch geeignet sind und bei deren Verwendung nur ein geringer Apparateaufwand notwendig ist.

Es bedarf keiner separat aufzustellender Pumpen und entsprechender Absaug- und Spülbehälter, weil alle Teile für das Spülen und Absaugen von Wundgebieten in ein und dem gleichen handbetätigten Gerät vereinigt sind.

### Zeichnungslegende

- 1: Saug-Druckpumpe
- 2: Gehäuse
- 3: Saugpumpe
- 4: Stößel
- 5: Ventilblock
- 6: Auffangbehälter
- 7: Gewinde
- 8: Dichtung
- 9: Schlauch
- 10: Öffnung
- 11: Atmungsventil
- 12: Spül-Saugrohr
- 13: Spülpumpe
- 14: Stößel
- 15: Ventilblock
- 16: Spülbehälter
- 17: Gehäuseschale
- 18: Hebel
- 19: Feder
- 20: Schieber
- 21: Ansatz
- 22: Ventilkammer
- 23: Ventilkammer
- 24: Ventil
- 25: Ventil
- 26: Anschluss
- 27: Anschluss
- 28: Zylinder
- 29: Kolben
- 30: Feder
- 31: Anschluss
- 32: Pfeilrichtung
- 33: Einsatz
- 34: Ventilkammer
- 35: Ventilkammer
- 36: Pfeilrichtung
- 37: Anschluss
- 38: Anschluss
- 39: Zylinder
- 40: Kolben
- 41: Feder
- 42: Anschluss
- 43: Querteil
- 44: Bohrung
- 45: Bohrung
- 46: Bohrung (Schieber)
- 47: Bohrung (Schieber)
- 48: Pfeilrichtung
- 49: Rastnase
- 50: Ausnehmung
- 51: Saugrohr
- 52: Spülrohr
- 53: Spülfächer
- 54: Entlastungsbohrung
- 55: Ansaugbohrung
- 56: Spüldüse
- 57: Spülrohr
- 58: Stößel

## Patentansprüche

1. Medizinisches Spül- und Sauggerät zum Spülen und Absaugen von Körperflächen in einem Operationsgebiet, bestehend aus einer Spül- und Absauglanze und einer damit verbundenen Ventilanordnung mit umschaltbaren Ventilen, welche je nach Ventilstellung eine Spülflüssigkeit der Spül- und Absauglanze zuführen oder diese in Absaugfunktion schalten, **dadurch gekennzeichnet, dass** das Spül- und Sauggerät (1) alle Funktionsteile in einem handhaltbaren Gerät vereinigt.

2. Medizinisches Spül- und Sauggerät nach Anspruch 1, **dadurch gekennzeichnet, dass** an einer handbetätigten Saug- und Spülpumpe (3,13) jeweils mindestens ein Spülbehälter und getrennt davon ein Auffangbehälter angeordnet sind.

3. Medizinisches Spül- und Sauggerät nach Anspruch 1 oder 2, **dadurch gekennzeichnet, dass** das Gerät (1) die zur Erzeugung eines Spülflüssigkeitsstromes erforderliche Spülpumpe (13) und gleichzeitig auch getrennt davon eine für das Absaugen aus dem Wundgebiet notwendige Absaugpumpe (3) enthält.

4. Medizinisches Spül- und Sauggerät nach Anspruch 3, **dadurch gekennzeichnet, dass** jeder Pumpe (3,13) ein eigener Behälter (6,16) zugeordnet ist.

5. Medizinisches Spül- und Sauggerät nach Anspruch 4, **dadurch gekennzeichnet, dass** mindestens der Auffangbehälter (6) abnehmbar und nach dem Abnehmen auch verschließbar ist.

6. Medizinisches Spül- und Sauggerät nach einem der Ansprüche 1 bis 5, **dadurch gekennzeichnet, dass** auch der Spülbehälter (16) abnehmbar und auswechselbar an der Saug- und Druckpumpe angeordnet ist.

7. Medizinisches Spül- und Sauggerät nach einem der Ansprüche 1 bis 5, **dadurch gekennzeichnet, dass** der Spülbehälter (16) als nicht-abnehmbar im Gerät (1) integriert ist.

8. Medizinisches Spül- und Sauggerät nach einem der Ansprüche 1 bis 7, **dadurch gekennzeichnet, dass** beiden Pumpen (3,13) nur wechselseitig betätigbar sind.

9. Medizinisches Spül- und Sauggerät nach Anspruch 8, **dadurch gekennzeichnet, dass** der wechselseitige Betrieb der Pumpen (3,13) durch einen Schieber (20) erfolgt, der handbetätigten Hebel (18) der Saug-Druckpumpe (1) angeordnet ist.

10. Medizinisches Spül- und Sauggerät nach einem der Ansprüche 1 bis 9, **dadurch gekennzeichnet, dass** das Spül-Saugrohr (12) zwei getrennt voneinander angeordnete Kanäle (51,52) für Spülung und Absaugung aufweist.

11. Medizinisches Spül- und Sauggerät nach einem der Ansprüche 1 bis 10, **dadurch gekennzeichnet, dass** zur Betätigung der Pumpen (3,13) ein einseitig schwenkbarer, federbelasteter Hebel (18) im Gehäuse (2) angeordnet ist, der mit einem Querteil (53) verbunden ist, in dem zwei voneinander beabstandete Bohrungen (44, 45) angeordnet sind, die wahlweise von den zugeordneten Bohrungen (46,47) von einem im Querteil (53) verschiebbar geführten Schieber (20) in Überdeckung oder Abdeckung bringbar sind, wobei die Stößel (4,58) der beiden Pumpen (3,13) jeweils fluchtend zu den Bohrungen (44,45) im Querteil (53) ausgerichtet sind.

## Geänderte Patentansprüche

### Geänderte Patentansprüche gemäss Regel 86(2) EPÜ.

**1.** Medizinisches Spül- und Sauggerät zum Spülen und Absaugen von Körperflächen in einem Operationsgebiet, bestehend aus einer Spül- und Absauglanze und einer damit verbundenen Ventilanordnung mit umschaltbaren Ventilen, welche je nach Ventilstellung eine Spülflüssigkeit der Spül- und Absauglanze zuführen oder diese in Absaugfunktion schalten und das Spül- und Sauggerät (1) alle Funktionsteile in einem handhaltbaren Gerät vereinigt, **dadurch gekennzeichnet, dass** zur Betätigung der Pumpen (3,13) ein einseitig schwenkbarer, federbelasteter Hebel (18) im Gehäuse (2) angeordnet ist, der mit einem Querteil (53) verbunden ist, in dem zwei voneinander beabstandete Bohrungen (44,45) angeordnet sind, die wahlweise von den zugeordneten Bohrungen (46,47) von einem im Querteil (53) verschiebbar geführten Schieber (20) in Überdeckung oder Abdeckung bringbar sind, wobei die Stößel (4,58) der beiden Pumpen (3,13) jeweils fluchtend zu den Bohrungen (44,45) im Querteil (53) ausgerichtet sind.

**2.** Medizinisches Spül- und Sauggerät nach Anspruch 1, **dadurch gekennzeichnet, dass** an einer handbetätigten Saug- und Spülpumpe (3,13) jeweils mindestens ein Spülbehälter und getrennt davon ein Auffangbehälter angeordnet sind.

**3.** Medizinisches Spül- und Sauggerät nach Anspruch 1 oder 2, **dadurch gekennzeichnet, dass** das Gerät (1) die zur Erzeugung eines Spülflüssigkeitsstromes erforderliche Spülpumpe (13) und gleichzeitig auch getrennt davon eine für das Absaugen aus dem Wundgebiet notwendige Absaugpumpe (3) enthält.

**4.** Medizinisches Spül- und Sauggerät nach Anspruch 3, **dadurch gekennzeichnet, dass** jeder Pumpe (3,13) ein eigener Behälter (6,16) zugeordnet ist.

**5.** Medizinisches Spül- und Sauggerät nach Anspruch 4, **dadurch gekennzeichnet, dass** mindestens der Auffangbehälter (6) abnehmbar und nach dem Abnehmen auch verschließbar ist.

**6.** Medizinisches Spül- und Sauggerät nach einem der Ansprüche 1 bis 5, **dadurch gekennzeichnet, dass** auch der Spülbehälter (16) abnehmbar und auswechselbar an der Saug- und Druckpumpe angeordnet ist.

**7.** Medizinisches Spül- und Sauggerät nach einem der Ansprüche 1 bis 5, **dadurch gekennzeichnet, dass** der Spülbehälter (16) als nicht-abnehmbar im Gerät (1) integriert ist.

**8.** Medizinisches Spül- und Sauggerät nach einem der Ansprüche 1 bis 7, **dadurch gekennzeichnet, dass** beiden Pumpen (3,13) nur wechselseitig betätigbar sind.

**9.** Medizinisches Spül- und Sauggerät nach Anspruch 8, **dadurch gekennzeichnet, dass** der wechselseitige Betrieb der Pumpen (3,13) durch einen Schieber (20) erfolgt, der handbetätigten Hebel (18) der Saug-Druckpumpe (1) angeordnet ist.

**10.** Medizinisches Spül- und Sauggerät nach einem der Ansprüche 1 bis 9, **dadurch gekennzeichnet, dass** das Spül-Saugrohr (12) zwei getrennt voneinander angeordnete Kanäle (51,52) für Spülung und Absaugung aufweist.
